(19) Europäisches Patentamt
European Patent Office
Office européen des brevets

(11) Veröffentlichungsnummer : **0 046 950**
**B1**

(12) # EUROPÄISCHE PATENTSCHRIFT

(45) Veröffentlichungstag der Patentschrift : **11.04.84**

(51) Int. Cl.$^3$ : **C 07 C121/75**, A 01 N 53/00

(21) Anmeldenummer : **81106531.7**

(22) Anmeldetag : **22.08.81**

(54) **Optisch aktive Isomere von trans-3-(2-Chlor-2-(4-chlor-phenyl)-vinyl)-2,2-dimethyl-cyclopropan-1-carbonsäure-(alpha-cyano-4-fluor-3-phenoxy-benzyl)-ester, Verfahren zu deren Herstellung und deren Verwendung als Ektoparasitizide.**

(30) Priorität : **03.09.80 DE 3033158**

(43) Veröffentlichungstag der Anmeldung : **10.03.82 Patentblatt 82/10**

(45) Bekanntmachung des Hinweises auf die Patenterteilung : **11.04.84 Patentblatt 84/15**

(84) Benannte Vertragsstaaten : **AT BE CH DE FR GB IT LI NL**

(56) Entgegenhaltungen :
**EP-A- 0 000 345**
**EP-A- 0 025 542**
**Van Valkenburg, Pestizide formulations (1973), S. 38-42**
**Soderlund et al., Pestizide Biochemistry and Physiology 7, 391-401 (1977)**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Fuchs, Rainer, Dr.**
**Roeberstrasse 8**
**D-5600 Wuppertal 1 (DE)**
Erfinder : **Stendel, Wilhelm, Dr.**
**In den Birken 55**
**D-5600 Wuppertal 1 (DE)**

**EP 0 046 950 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Optisch aktive Isomere von trans-3-(2-Chlor-2-(4-chlorphenyl)-vinyl)-2,2-dimethyl-cyclopropan-1-carbonsäure-(α-cyano-4-fluor-3-phenoxy-benzyl)-ester, Verfahren zu deren Herstellung und deren Verwendung als Ektoparasitizide

Die Erfindung betrifft neue optisch aktive Isomere von trans-3-(2-Chlor-2-(4-chlor-phenyl)vinyl)-2,2-dimethyl-cyclopropan-1-carbonsäure-(α-cyano-4-fluor-3-phenoxy-benzyl)-ester, Verfahren zu deren Herstellung und deren Verwendung als Ektoparasitizide.

Es ist bereits bekannt geworden, daß Gemische der (±)-cis- und (±)-trans-Formen von 3-(E/Z-2-Chlor-2-(4-chlorphenyl)-vinyl)-2,2-dimethyl-cyclopropan-1-carbonsäure- (±) -(α-cyano-4-fluor-3-phenoxy-benzyl)-ester insektizid und akarizid wirksam sind (vgl. DE-OS 2 730 515 Beispiel 17).

Weiter sind racemische Isomerengemische von trans-3-(2-Chlor-2-(4-chlor-phenyl)-vinyl)-2,2-dimethyl-cyclopropan-1-carbonsäure-(α-cyano-4-fluor-3-phenoxy-benzyl)-ester Gegenstand der älteren, nicht vorveröffentlichten europäischen Patentanmeldung 0 025 542.

Die vorliegende Erfindung betrifft

(1) optisch aktive Isomere von trans-3-(2-Chlor-2-(4-chlor-phenyl)-vinyl)-2,2-dimethyl-cyclopropan-1-carbonsäure-α-cyano-4-fluor-3-phenoxy-benzyl)-ester der allgemeinen Formel I

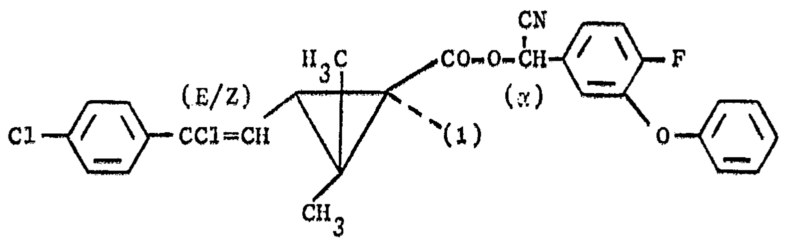

(I)

in der

(a) die Konfiguration am Cyclopropan-C-Atom (1) R oder S (Variable 1 = [1] ist, d. h. die den Verbindungen der Formel (I) zu Grunde liegenden Säuren drehen die Ebene des linear polarisierten Lichts nach rechts (+) bzw. nach links (−).

(b) die Konfiguration and der C-C-Doppelbindung E und/oder Z (Variable 2 = [2]) ist und

(c) die Konfiguration am α-C-Atom R und/oder S (Variable 3 = [3]) ist, gekennzeichnet durch die folgenden Variablenkombinationen :

| Isomeres | Variable 1(*) | Variable 2 (**) | Variable 3 (**) |
|---|---|---|---|
| (1) | (+) | E/Z | R/S |
| (2) | (+) | E | R/S |
| (3) | (+) | Z | R/S |
| (4) | (+) | E/Z | S |
| (5) | (+) | E | S |
| (6) | (+) | Z | S |
| (7) | (−) | E/Z | R/S |
| (8) | (−) | E | R/S |
| (9) | (−) | Z | R/S |
| (10) | (−) | E/Z | S |
| (11) | (−) | E | S |
| (12) | (−) | Z | S |

(*) Die Symbole (+) bzw. (−) beziehen sich auf die Richtung der Drehung des linear polarisierten Lichts durch die den Verbindungen der Formel (I) zu Grunde liegenden Säuren ; die entsprechenden absoluten Konfigurationen (R bzw. S) am Cyclopropangerüst sind nicht bekannt, die optisch aktiven Säuren sind jedoch durch die Angabe der Drehung des linear polarisierten Lichts eindeutig charakterisiert.

(**) Die Symbole E und Z bzw. R und S stehen für Verbindungen der Formel (I), worin die Isomeren der jeweils genannten Konfiguration auf mehr als 60 %, vorzugsweise auf mehr als 90 % angereichert sind ; E/Z und R/S stehen für Verbindungen der Formel (I), worin die Isomeren angenähert im Verhältnis 1 : 1, d. h. zwischen 60 : 40 und 40 : 60 vorliegen.

Die Isomeren (1) bis (6) sind vorzugsweise Gegenstand dieser Erfindung.

Die neuen, oben definierten Verbindungen der Formel (I) zeichnen sich durch hohe ektoparasitizide Wirksamkeit aus.

Ein weiterer Gegenstand der Patentanmeldung ist

(2) ein Verfahren zur Herstellung von Verbindungen der Formel (I), dadurch gekennzeichnet, daß man entsprechende optisch aktive Isomere von trans-3-(2-Chlor-2-(4-chlor-phenyl)-vinyl)-2,2-dimethyl-cyclopropan-1-carbonsäurechlorid der Formel II

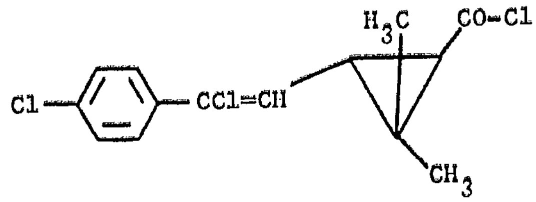

(II)

mit gegebenenenfalls entsprechenden optischen Isomeren von α-Cyano-4-fluor-3-phenoxy-benzylalkohol der Formel III

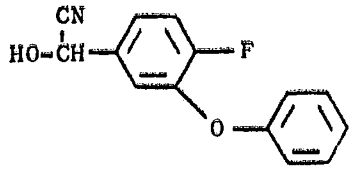

(III)

gegebenenfalls in Gegenwart eines Säureakzeptors und/oder in Gegenwart eines Verdünnungsmittels umsetzt oder, für den Fall, daß die Konfiguration am α-C-Atom R + S (ca. 1 : 1) sein soll, mit 4-Fluor-3-phenoxy-benzaldehyd der Formel IV

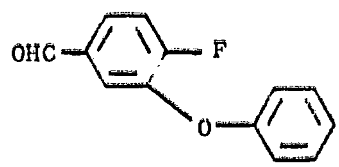

(IV)

in Gegenwart von wenigstens der äquimolaren Menge eines Alkalicyanids, gegebenenfalls in Gegenwart eines Katalysators und gegebenenfalls unter Verwendung von Verdünnungsmitteln umsetzt.

Überraschenderweise zeigen die neuen unter (1) definierten Verbindungen der Formel (I), insbesondere die Isomeren der Konfiguration (1) und (3), eine erheblich höhere insektizide und akarizide, insbesondere ektoparasitizide Wirkung als aus dem Stand der Technik bekannte Isomerengemische von 3-(2-Chlor-2-(4-chlorphenyl)-vinyl)-2,2-dimethyl-cyclopropan-1-carbonsäure-(α-cyano-4-fluor-3-phenoxy-benzyl)-ester.

Verwendet man als Ausgangsstoffe beispielsweise (+)-trans-3-(Z-2-Chlor-2-(4-chlor-phenyl)-vinyl)-2,2-dimethyl-cyclopropan-1-carbonsäurechlorid und (S)-α-Cyano-4-fluor-3-phenoxy-benzylalkohol bzw. 4-Fluor-3-phenoxy-benzaldehyd und Natriumcyanid, so können die entsprechenden Reaktionen durch folgendes Formelschema skizziert werden :

(a)

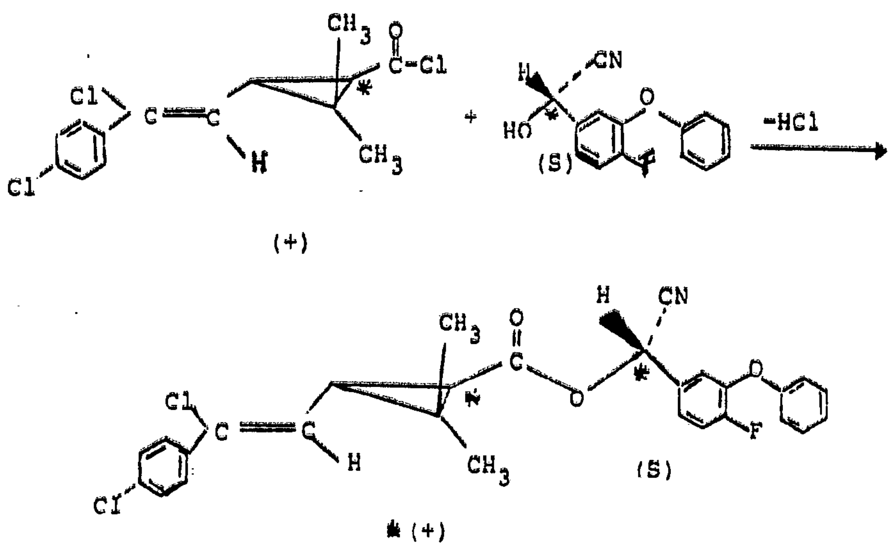


(+)

* (+)

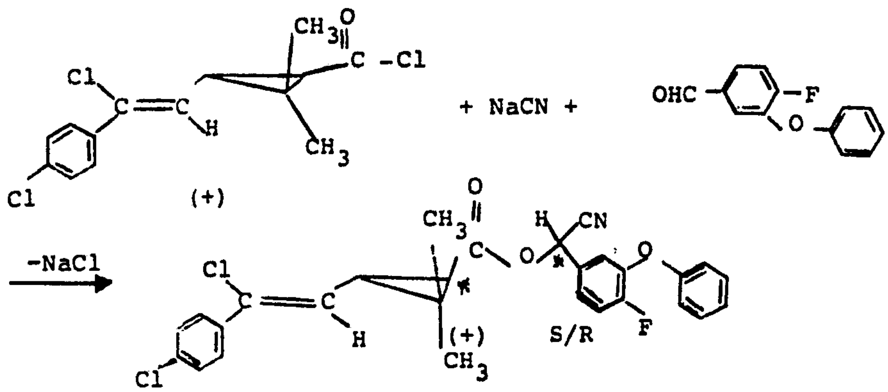


Das nach Verfahren (6) erhältliche Diastereomerengemisch aus (+)-trans-Z-3-(2-Chlor-2-(4-chlorphenyl)-vinyl)-2,2-dimethyl-cyclopropancarbonsäure-(S)-α-cyano-3-phenoxy-4-fluorbenzylester und (+)-trans-Z-3-(2-Chlor-2-(4-chlor-phenyl)-vinyl)-2,2-dimethyl-cyclopropancarbonsäure-(R)-α-cyano-3-phenoxy-4-fluorbenzylester läßt sich nach bekannten chromatographischen Methoden, z. B. Hochdruckflüssigkeitschromatographie, oder durch fraktionierte Kristallisation, in die beiden Einzelkomponenten auftrennen.

Die als Ausgangsstoffe zu verwendenden optisch aktiven Isomeren von trans-3-(2-Chlor-2-(4-chlor-phenyl)-vinyl)-2,2-dimethyl-cyclopropan-1-carbonsäurechlorid der Formel (II) sind noch nicht in der Literatur beschrieben.

Als Beispiele seien genannt :

(+)-trans-3-(E/Z-2-Chlor-2-(4-chlor-phenyl)-vinyl)-2,2-dimethyl-cyclopropan-1-carbonsäurechlorid,
(+)-trans-3-(E-2-Chlor-2-(4-chlor-phenyl)-vinyl)-2,2-dimethyl-cyclopropan-1-carbonsäurechlorid und
(+)-trans-3-(Z-2-Chlor-2-(4-chlor-phenyl)-vinyl)-2,2-dimethyl-cyclopropan-1-carbonsäurechlorid
sowie die entsprechenden linksdrehenden verbindungen.

Man erhält die neuen Verbindungen der Formel (II), wenn man die entsprechenden optisch aktiven Isomeren von trans-3-(2-Chlor-2-(4-chlor-phenyl)-vinyl)-2,2-dimethyl-cyclopropan-1-carbonsäure der Formel V

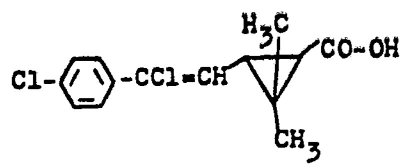

(V)

mit einem Chlorierungsmittel, wie z. B. Thionylchlorid, gegebenenfalls in Gegenwart eines Verdünnungsmittels, wie z. B. Tetrachlorkohlenstoff, bei Temperaturen zwischen 10 und 100 °C umsetzt.

Optisch aktive Isomere von trans-3-(2-Chlor-2-(4-chlorphenyl)-vinyl)-2,2-dimethyl-cyclopropan-1-carbonsäure (V) sind noch nicht in der Literatur beschrieben.

Als Beispiele seien genannt :

(+)-trans-3-(E/Z-2-Chlor-2-(4-chlor-phenyl)-vinyl)-2,2-dimethyl-cyclopropan-1-carbonsäure,
(+)-trans-3-(E-2-Chlor-2-(4-chlor-phenyl)-vinyl)-2,2-dimethyl-cyclopropan-1-carbonsäure und
(+)-trans-3-(Z-2-Chlor-2-(4-chlor-phenyl)-vinyl)-2,2-dimethyl-cyclopropan-1-carbonsäure
sowie die entsprechenden linksdrehenden Isomeren.

Man kann die optisch aktiven Isomeren von trans-3-(2-Chlor-2-(4-chlor-phenyl)-vinyl)-2,2-dimethyl-cyclopropan-1-carbonsäure aus racemischen Gemischen von (V) nach üblichen Anreicherungs- bzw. Trennmethoden erhalten (vgl. DE-OS 2 826 952). Diese Trennungen bzw. Anreicherungen erfolgen im allgemeinen durch fraktionierte Ausfällung bzw. Kristallisation der Salze mit optisch aktiven Aminen bzw. der Ester mit optisch aktiven Alkoholen in die jeweiligen optisch aktiven Diastereomeren ; ein spezielles Verfahren ist weiter unten beschrieben.

Die Herstellung racemischer Gemische der diastereomeren Formen von trans-3-(2-Chlor-2-(4-chlor-phenyl)-vinyl)-2,2-dimethyl-cyclopropan-1-carbonsäure ist Gegenstand der älteren, nicht vorveröffentlichen europäischen Patentanmeldung 0 025 542.

(±)-trans-3-(E,Z-2-Chlor-2-(4-chlor-phenyl)-vinyl)-2,2-dimethyl-cyclopropan-1-carbonsäure der Formel (V) erhält man durch Verseifung von entsprechenden Alkylestern der Formel VI

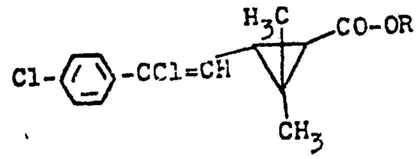

(VI)

in welcher

R für $C_1$-$C_4$-Alkyl steht, nach üblichen Methoden, beispielsweise, indem man die Ester mit Alkalilaugen, wie z. B. wässrig-alkoholischer Natronlauge auf Temperaturen zwischen 50 und 100 °C erhitzt. Zur Aufarbeitung wird gegebenenfalls der Alkohol abdestilliert und das Produkt mit einem mit Wasser nicht mischbaren Lösungsmittel, wie z. B. Methylenchlorid, extrahiert und das Extraktionsmittel unter vermindertem Druck abdestilliert.

Als Beispiele für die Ester der Formel (VI) seien genannt:

(±)-trans-3-(E,Z-2-Chlor-2-(4-chlor-phenyl)-vinyl)-cyclopropan-1-carbonsäure-methylester, -ethylester, -n-propylester, -iso-propylester, -n-butylester, -iso-butylester, -sekt.-butylester und -tert.-butylester.

Die Ester der Formel (VI) sind noch nicht in der Literatur beschrieben. Man erhält sie, indem man (±)-trans-3-Formyl-2,2-dimethyl-cyclopropan-1-carbonsäureester der Formel VII

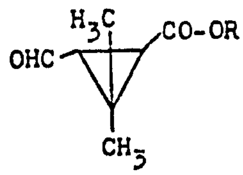

(VII)

in welcher

R für $C_1$-$C_4$-Alkyl steht, mit 4-Chlor-α-chlor-benzyl-phosphonsäureestern der Formel VIII

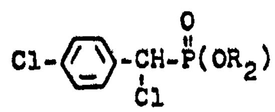

(VIII)

in welcher

R für $C_1$-$C_4$-Alkyl steht, in Gegenwart von Basen, wie z. B. Natriummethylat, gegebenenfalls in Gegenwart von Verdünnungsmitteln, wie z. B. Ethanol und/oder Tetrahydrofuran, bei Temperaturen zwischen − 10 und + 50 °C umsetzt. Zur Aufarbeitung wird mit Wasser verdünnt und mit einem mit Wasser nicht mischbaren Lösungsmittel, wie z. B. Methylenchlorid, extrahiert. Die Extrakte werden getrocknet, filtriert und das Lösungsmittel wird unter Vermindertem Druck abdestilliert.

Ester der Formel (VII) sind bereits bekannt (vgl. DE-OS 2 615 160). Als Beispiele seien genannt:

(±)-trans-3-Formyl-2;2-dimethyl-cyclopropan-1-carbonsäure-methylester, -ethylester, -n-propylester, -isopropylester, -n-butylester, -iso-butylester, -sek.-butylester und -tert.-butylester.

4-Chlor-α-chlor-benzyl-phosphonsäureester der Formel (VIII) sind ebenfalls bereits bekannt (vgl. DE-OS 2 827 101). Als Beispiele seien genannt:

4-Chlor-α-chlor-benzyl-phosphonsäure-dimethylester und -diethylester.

Neben optisch aktiven Isomeren von trans-3-(2-Chlor-2-(4-chlor-phenyl)-vinyl)-2,2-dimethyl-cyclopropan-1-carbonsäurechlorid (II) sind die einzelnen Enantiomeren bzw. das racemische Gemisch von α-Cyano-4-fluor-3-phenoxy-benzylalkohol der Formel (III) bzw. 4-Fluor-3-phenoxy-benzaldehyd der Formel (IV) als Ausgangsstoffe bei der Herstellung der unter (1) definierten neuen Verbindungen der Formel (I) zu verwenden.

4-Fluor-3-phenoxy-benzaldehyd (IV) und racemische Gemische von α-Cyano-4-fluor-3-phenoxy-benzylalkohol (III) sind bereits bekannt (vgl. DE-OS 2 709 264).

Die einzelnen Enantiomeren aus (III) können nach üblichen Methoden getrennt werden (vgl. DE-OS 2 902 466).

Alkalicyanide, welche zur Herstellung der neuen Verbindungen der Formel (I) zu verwenden sind, sind vorzugsweise Natriumcyanid und Kaliumcyanid.

Das Verfahren zur Herstellung der neuen Verbindungen der Formel (I) wird vorzugsweise unter Verwendung von Verdünnungsmitteln durchgeführt. Als solche kommen praktisch alle inerten organischen Solventien infrage. Hierzu gehören insbesondere aliphatische und aromatische, gegebenenfalls chlorierte Kohlenwasserstoffe, wie Pentan, Hexan, Heptan, Cyclohexan, Benzol, Toluol, Xylol, Methylenchlorid, Chloroform, Tetrachlorkohlenstoff, Chlorbenzol und o-Dichlorbenzol, Ether wie Diethyl- und Dibutylether, Tetrahydrofuran, und Dioxan, Ketone wie Aceton, Methylethyl-, Methyl-iso-propyl- und Methylisobutylketon sowie Nitrile wie Acetonitril und Propionitril.

Bei Verwendung von 4-Fluor-3-phenoxy-benzaldehyd und Natriumcyanid werden vorzugsweise von den oben genannten Lösungsmitteln die mit Wasser nicht mischbaren in Kombination mit Wasser als

zweiter Solvenskomponente verwendet, d. h. das Verfahren wird in einem zweiphasigen Medium durchgeführt. Hierbei können als Katalysatoren Verbindungen verwendet werden, welche gewöhnlich bei Reaktionen in mehrphasigen Medien als Hilfsmittel zum Phasentransfer von Reaktanden dienen. Insbesondere seien Tetraalkyl- und Trialkyl-aralkyl-ammoniumsalze, wie z. B. Tetrabutylammoniumbromid, Methyltrioctylammoniumchlorid und Trimethylbenzylammoniumhydrogensulfat genannt.

Die Reaktionstemperatur wird im allgemeinen zwischen 0 und 100 °C, vorzugsweise zwischen 10 und 50 °C gehalten. Das Herstellungsverfahren wird gewöhnlich bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in geeigneten Verdünnungsmitteln gegebenenfalls in Gegenwart eines Katalysators durchgeführt und das Reaktionsgemisch wird mehrere Stunden bei der erforderlichen Temperatur gerührt. Danach gibt man ein organisches Lösungsmittel wie z. B. Toluol, zu und arbeitet die organische Phase wie üblich durch Waschen, Trocknen und Abdestillieren des Lösungsmittels auf.

Die neuen Verbindungen der Formel (I) fallen in öliger Form an und können nicht unzersetzt destilliert werden, jedoch durch sogenanntes « Andestillieren », d. h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der $(\alpha)_D$-Wert.

E/Z-Isomerengemische der (±)-trans-3-(E/Z-2-Chlor-2-(4-chlor-phenyl)-vinyl)-2,2-dimethyl-cyclopropan-1-carbonsäure der Formel V (oben) kann man trennen, indem man Isomerengemische der Säure (V) in einem mit Wasser nicht mischbaren Lösungsmittel, wie z. B. Methylenchlorid, löst, durch Zugabe von 0,001 bis 1, vorzugsweise 0,1 bis 1 Äquivalenten einer Base, wie z. B. wässriger Natronlauge, in X Schritten, wobei X für eine Zahl zwischen 1 und ∞, wobei ∞ eine kontinuierliche Arbeitsweise bedeutet, vorzugsweise aber für 2 bis 100 steht, in das Salz überführt, nach jeder Basenzugabe das Salz mit Wasser in X Schritten extrahiert, die einzelnen wässrigen Fraktionen mit einer Mineralsäure, wie z. B. Salzsäure ansäuert, mit einem mit Wasser nicht mischbaren Lösungsmittel, wie z. B. Methylenchlorid, extrahiert, jeden einzelnen Extrakt nach üblichen Methoden, beispielsweise durch Trocknen, Filtrieren und Abdestillieren des Lösungsmittels aufarbeitet und die Fraktionen, in denen eines der Isomeren (Z oder E) nach NMR-spektroskopischer Analyse überwiegt, aus einem organischen Lösungsmittel, vorzugsweise einem Kohlenwasserstoff mit 5 bis 10 Kohlenstoffatomen, umkristallisiert.

Eine weitere Trennmethode für die E/Z-Säuregemische besteht darin, daß man Isomerengemische der Säure (V) in einer wässrigen Alkalilauge, wie z. B. Natronlauge, löst, welche genau ein Basenäquivalent enthält, durch Zugabe von 0,001 bis 1, vorzugsweise von 0,1 bis 1 Äquivalenten einer Mineralsäure in X Schritten, wobei X für eine Zahl zwischen 1 und ∞, wobei ∞ eine kontinuierliche Arbeitsweise bedeutet, vorzugsweise aber zwischen 2 und 100 steht, wieder die Säure erzeugt, nach jeder Säurezugabe mit einem mit Wasser nicht mischbaren organischen Lösungsmittel, wie z. B. Methylenchlorid, extrahiert, jeden einzelnen Extrakt nach üblichen Methoden, beispielsweise durch Trocknen, Filtrieren und Abdestillieren des Lösungsmittels, aufarbeitet und die Fraktionen, in denen eines der Isomeren (Z oder E) nach NMR-spektroskopischer Analyse überwiegt, aus einem organischen Lösungsmittel, vorzugsweise einem Kohlenwasserstoff mit 5 bis 10 Kohlenstoffatomen, umkristallisiert. (±)-Diastereomerengemische von E/Z, E- oder Z-trans-3-(2-Chlor-2-(4-chlor-phenyl)-vinyl)-2,2-dimethyl-cyclopropan-1-carbonsäure der Formel V (oben) können, wie bereits erwähnt, nach üblichen Methoden getrennt werden (vgl. DE-OS 2 826 952).

Man kann solche Gemische beispielsweise trennen, indem man sie, oder entsprechende Alkalisalze, mit D-(−)-threo 2-Amino-1-(4-nitro-phenyl)-1,3-propandiol oder dessen Hydrochlorid in Wasser/Alkohol-Gemischen bis zur praktisch vollständigen Auflösung auf Temperaturen zwischen 50 und 100 °C erhitzt und die Lösungen langsam auf Temperaturen zwischen 0 und 30 °C abkühlen läßt. Hierbei kristallisieren an den (+)-Isomeren angereicherte Salze aus, während die Slaze der (−)-Isomeren überwiegend in Lösung bleiben. Nach Trennung durch Filtration — das (+)-Isomere kann dann durch Umkristallisation noch stärker angereichert werden — können aus den im Kristallisat bzw. in der Mutterlauge befindlichen, an den einzelnen Diastereomeren angereicherten Salzen die entsprechenden Säuren durch Ansäuern mit starken Säuren freigesetzt werden. Beispielsweise wird das Kristallisat, welches vorwiegend das Salz aus (+)-trans-Z-3-(2-Chlor-2-(4-chlor-phenyl)-vinyl)-2,2-dimethyl-cyclopropan-1-carbonsäure und D-(−)-threo-2-Amino-1-(4-nitro-phenyl)-1,3-propandiol enthält, in einem Zweiphasensystem aus Methylenchlorid und verdünnter Salzsäure dispergiert und das Gemisch wird einige Minuten intensiv gerührt. Anschließend wird die organische Phase abgetrennt, getrocknet und filtriert. Vom Filtrat wird das Lösungsmittel unter vermindertem Druck sorgfältig abdestilliert, wobei man als Rückstand (+)-trans-Z-3-(2-Chlor-2-(4-chlorphenyl)-vinyl)-2,2-dimethyl-cyclopropan-1-carbonsäure erhält.

Die erfindungsgemäßen Wirkstoffe eignen sich zur Bekämpfung von Ektoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and sport-on) und des Einpuders sowie durch parenterale Anwendung in Form beispieleswelse der Injektion.

## Herstellungsbeispiele

### Beispiel 1

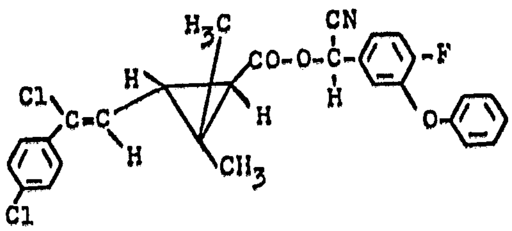

Zu einer Mischung von 50 ml Cyclohexan, 0,87 g Natriumcyanid, 1,3 ml Wasser, 2,42 g (0,011 2 Mol) 3-Phenoxy-4-fluorbenzaldehyd und 0,2 g Tetrabutylammoniumbromid werden unter Rühren bei 20-25 °C 3,4 g (0,011 2 Mol) (+)-trans-Z-3-(2-Chlor-2-(4-chlor-phenyl)-vinyl)-2,2-dimethyl-1-cyclopropancarbonsäurechlorid gelöst in 10 ml Cyclohexan zugetropft und dann 4 Stunden bei 20-25 °C gerührt. Anschließend wird die Reaktionsmischung mit 100 ml Toluol versetzt und 2 mal mit je 60 ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 60 °C/l Torr Badtemperatur entfernt. Man erhält 4,4 g (77 % der Theorie) (+)-trans-Z-3-(2-Chlor-2-(4-chlorphenyl)-vinyl)-2,2-dimethyl-cyclopropancarbonsäure-(±)-α-cyano-3-phenoxy-4-fluor-benzylester als zähes Öl, mit der spezifischen Drehung $[\alpha]_D = + 58,2°$ (C = 100 mg in 10 ml $CHCl_3$).

$^1$H-NMR-Spektrum in $CDCl_3$/TMS τ (ppm).
Benzyl-H : 3,60 (S/1/2H) und 3,64 (S/1/2H).
Vinyl-H : 4,12 (d/1H).

Trennung der α-R und α-S- Diastereomeren

5 g of (+)-trans-Z-3-(2-Chloro-2-(4-chloro-phenyl)-vinyl)-2,2-dimethyl-cyclopropane-carbonsäure (RS)-α-cyano-3-phenoxy-4-fluoro-benzylester werden der präparativen HPLC-Methode (high pressure liquid chromatography) unterzogen.

Säule : 250 mm × 21,2 mm, 7 μ Silicagel.
Mobile Phase : 47,6 Vol.% n-Hexan, 47,6 Vol. % Cyclohexan und 4,8 Vol.% Diäthyläther.
Durchflußmenge : 30 ml/min.
Angewendete Menge : 30 mg.
Retentionsvolumen : Fraktion I : 300 ml.
Fraktion II : 330 ml.

Anschließend wurden die beiden Fraktionen im Vakuum vom Lösungsmittel befreit.

Als Fraktion I wurden 0,9 g (+)-trans-Z-3-(2-Chloro-2-(4-chloro-phenyl)-vinyl)-2,2-dimethyl-cyclopropancarbonsäure-(R)-α-cyano-3-phenoxy-4-fluorobenzylester als farbloses Öl erhalten $[\alpha]^{20} = + 41,0$ °C (c = 190 mg in 10 ml $CHCl_3$).
$^1$H-NMR-Spektrum ($CDCl_3$/TMS) δ cppm) : —CHCN : 6,351 (S/1H)
Dimethyl-H : 1,37 (s/3H) und 1,26 (S/3H).

Als Fraktion II wurden 0,5 g (+)-trans-Z-3-(2-Chloro-2)-(4-chloro-phenyl)-vinyl)-2,2-dimethyl-cyclopropancarbonsäure-(S)-α-cyano-3-phenoxy-4-fluoro-benzylester als farbloses Öl erhalten.
$[\alpha]^{20} = + 34,5$ °C (c = 120 mg in 10 ml $CHCl_3$).
$^1$H-NMR-Spektrum ($CDCl_3$/TMS) α (ppm) :
—CHCH : 6,377 (S/1H).
Dimethyl-H : 1,28 (S/3H) and 1,22 (S/3H).

Beispiele zur Herstellung der Ausgangsverbindungen :

(+) 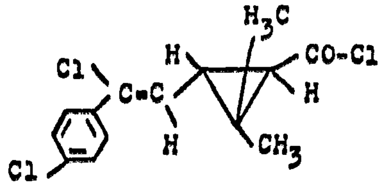

4,2 g (0,014 7 Mol) (+)-trans-Z-3-(2-Chlor-2-(4-chlor-phenyl)-vinyl)-2,2-dimethyl-1-cyclopropancarbonsäure werden in 50 ml Tetrachlorkohlenstoff gelöst und bei 40 °C langsam unter Rühren 8,8 g Thionylchlorid zugetropft. Anschließend wird 4 Stunden zum Rückfluß erhitzt. Nach beendeter

Reaktionszeit werden überschüssiges Thionylchlorid sowie Tetrachlorkohlenstoff im Wasserstrahlvakuum abdestilliert. Letzte Lösungsmittelreste werden durch kurzes Andestillieren bei 2 Torr/60 °C Badtemperatur entfernt. Man erhält 4,0 g (89,7 % der Theorie) (+)-trans-Z-(3-(2-Chlor-2-(4-chlor-phenyl)-vinyl)-2,2-dimethyl-cyclopropancarbonsäurechlorid als kristallinen Feststoff mit einem Schmelzpunkt von 65-70 °C.

(+) 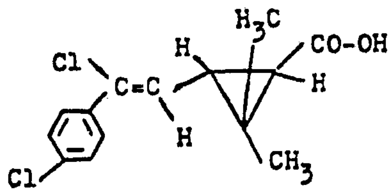

8,8 g (0,017 7 Mol) des Salzes der (+)-trans-Z-3-(2-Chlor-2-(4-chlor-phenyl)-vinyl)-2,2-dimethyl-1-cyclopropancarbonsäure mit dem D-(−)-threo-2-Amino-1-(4-nitro-phenyl)-1,3-propandiol werden in 100 ml Methylenchlorid suspendiert mit einer Mischung von 25 ml konz. Salzsäure in 100 ml Wasser versetzt und dann intensiv gerührt. Anschießend wird die organische Phase abgetrennt, über Magnesiumsulfat getrocknet und das Lösungsmittel im Wasserstrahlvakuum abdestilliert. Letzte Lösungsmittelreste werden durch kurzes Andestillieren bei 60 °C/l Torr Badtemperatur entfernt. Man erhält 4,5 g (89,2 % der Theorie) (+)-trans-Z-3-(2-Chlor-2-(4-chlor-phenyl)-vinyl)-2,2-dimethyl-1-cyclopropancarbonsäure als farblose Kristalle mit dem Schmelzpunkt von 105-106 °C und einer spezifischen Drehung von $[\alpha]_D = +122°$ (C = 100 mg in 10 ml $CHCl_3$).

(+) 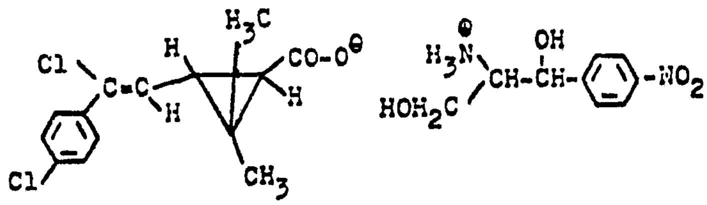 (-)

A) Eine Mischung von 2,85 g (0,01 Mol) (±)-trans-Z-3-(2-Chlor-2-(4-chlor-phenyl)-vinyl)-2,2-dimethyl-1-cyclopropancarbonsäure und 2,12 g (0,01 Mol) D-(−)-threo-2-Amino-1-(4-nitro-phenyl)-1,3-propandiol in 180 ml eines Gemisches aus 7 Vol. Teilen Wasser und 3 Vol. Teilen Ethanol wird unter Rührend solange auf 65-70 °C erhitzt, bis man eine klare Lösung erhält. Dann läßt man langsam auf Raumtemperatur (20 °C) abkühlen und anschließend noch 6 Stunden bei 20 °C stehen. Die ausgefallenen Kristalle werden abgesaugt und nochmals aus dem Wasser/Ethanol-Gemisch 7 : 3 umkristallisiert. Man erhält 0,92 g (37 % der Theorie) des Salzes der (+)-trans-Z-3-(2-Chlor-2-(4-chlor-phenyl)-vinyl)-2,2-dimethyl-cyclopropan-1-carbonsäure mit dem D-(−)-threo-2-Amino-1-(4-nitro-phenyl)-1,3-propandiol als farblose, perlmuttartige Blättchen mit dem Schmelzpunkt von 160-162 °C.

B) Eine Mischung von 28,5 g (0,1 Mol) (±)-trans-Z-(3-(2-Chlor-2-(4-chlor-phenyl)-vinyl)-2,2-dimethyl-cyclo-propan-1-carbonsäure, 540 ml Ethanol, 1 260 ml Wasser und 4 g (0,1 Mol) Natriumhydroxyd wird unter Rühren solange auf 70-75 °C erwärmt, bis eine klare Lösung entstanden ist. Zu dieser Mischung gibt man unter Rühren 12,5 g (0,05 Mol) D-(−)-threo-2-Amino-1-(4-nitro-phenyl)-1,3-propandiol-hydrochlorid, gelöst in 80 ml Wasser und erwärmt nochmals 10 Minuten auf 70-75 °C. Dann läßt man langsam ohne Rühren auf Raumtemperatur abkühlen und läßt dann noch 5 Stunden bei 20 °C stehen. Die ausgefallenen Kristalle werden abgesaugt und nochmals aus einem Wasser/Ethanol-Gemisch 7 : 3 umkristallisiert. Man erhält 8,8 g (35,4 % der Theorie) des Aminsalzes aus (+)-trans-Z-(3-(2-Chlor-2-(4-chlor-phenyl)-vinyl)-2,2-dimethyl-cyclopropan-1-carbonsäure und D-(−)-threo-2-Amino-1-(4-nitro-phenyl)-1,3-propandiol als farblose Kristalle mit dem Schmelzpunkt 157-160 °C.

(±) 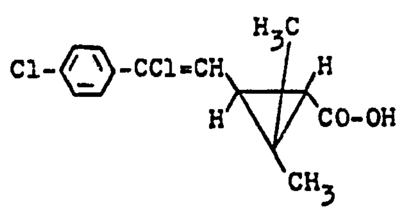

22,2 g (0,071 Mol) (±)-trans-3-(E,Z-2-Chlor-2-(4-chlor-phenyl)-vinyl)-2,2-dimethyl-cyclopropancarbonsäure-ethylester werden in 100 ml Ethanol gelöst, dann mit einer Lösung von 5,7 g Natriumhydroxid in 100 ml Wasser versetzt und 4 Stunden unter Rühren zum Rückfluß erhitzt. Anschließend wird das Ethanol im Wasserstrahlvakuum abdestilliert, der Rückstand in 300 ml warmen Wasser aufgenommen und 1 mal mit 300 ml Methylenchlorid extrahiert. Die wässrige Phase wird abgetrennt, mit konzentrierter Salzsäure angesäuert und anschließend mit 2 mal 300 ml Methylenchlorid extrahiert. Die organische Phase wird abgetrennt über Magnesiumsulfat, getrocknet und das Lösungsmittel im Wasserstrahlvakuum abdestilliert. Letzte Lösungsmittelreste werden durch kurzes Andestillieren bei 2 Torr/60 °C Badtemperatur entfernt. Man erhält 15,5 g (76,6 % der Theorie) (±)-trans-3-(E,Z-2-Chlor-2-(4-chlor-phenyl)-vinyl)-2,2-dimethyl-cyclopropancarbonsäure als zähes Öl, das nach einiger Zeit kristallisiert und aus Acetonitril umkristallisiert bei 120-132°C schmilzt.

(±) 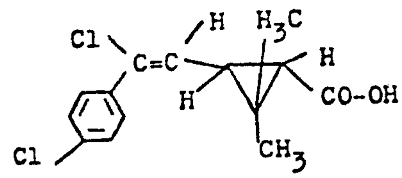

31,6 g (0,111 Mol) (±)-trans-(E,Z-2-Chlor-2-(4-chlor-phenyl)-vinyl)-2,2-dimethyl-cyclopropancarbonsäure mit einem E/Z-Verhältnis von 60 : 40, werden in 150 ml Methylenchlorid gelöst und mit einer Lösung von 0,885 g (0,022 Mol) Natriumhydroxid in 50 ml Wasser ausgeschüttelt. Anschließend wird die wässrige Phase abgetrennt.

Dieser Vorgang wird anschließend noch 4 mal wiederholt. Man erhält so 5 Fraktionen einer wässrigen Salzlösung, die anschließend jede für sich mit konzentrierter Salzsäure angesäuert und anschließend mit je 2 mal 50 ml Methylenchlorid extrahiert werden. Die organischen Phasen werden abgetrennt, über Magnesiumsulfat getrocknet und dann das Lösungsmittel im Vakuum abgezogen. Man erhält so 5 Fraktionen obiger Säure mit unterschiedlichem E/Z-Verhältnis. Das E/Z-Verhältnis wird durch das $^1$H-NMR-Spektrum bestimmt.

Fraktion I : d. h. die zuerst abgetrennte Säure ergibt ein E/Z-Verhältnis von 50/50.

Fraktion V : ein E/Z-Verhältnis von 87/13.

Fraktion V (5 g) wird anschließend bei 30-40 °C in Cyclohexan gelöst. Durch stehenlassen bei Raumtemperatur kristallisiert (±)-trans-3-(E-2-Chlor-2-(4-chlor-phenyl)-vinyl)-2,2-dimethyl-cyclopropancarbonsäure in Form farbloser Kristalle vom Schmelzpunkt 138-139 °C aus.

Die Struktur wird durch das $^1$H-NMR-Spektrum bestätigt.

$^1$H-NMR-Spektrum in $CDCl_3$/TMS, τ (ppm) :

aromatische-H : 2,43-2,74 (m/4 H), vinyl-H : 4,24 (d/1 H),
cyclopropan-H : 7,74-8,04 (m/1 H) und 8,39 (d/1 H),
dimethyl-H : 8,73 (s/6 H).

(±) 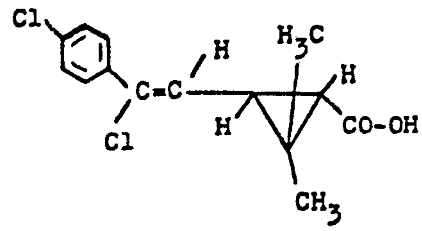

130,2 g (0,4568 Mol) (±)-trans-(E,Z-2-Chlor-2-(4-chlor-phenyl)-vinyl)-2,2-dimethyl-cyclopropancarbonsäure, mit einem E/Z-Verhältnis von 60/40, werden in 500 ml Wasser suspendiert und unter Rühren durch Zusatz von 18,27 g (0,456 8 Mol) Natriumhydroxid gelöst in 100 ml Wasser, in das Natriumsalz übergeführt. Die wässrige Salzlösung wird mit 100 ml Methylenchlorid unterschichtet und unter heftigem Rühren mit 0,045 68 Mol Chlorwasserstoff in Form einer 37 %igen wässrigen, Lösung, versetzt. Anschließend wird noch 5 Minuten gerührt und dann die Methylenchloridphase abgetrennt. Das Ansäuern und Abtrennen wird insgesamt noch 9 mal in der gleichen Weise wiederholt. Die 10 Methylenchloridphasen werden dann über Magnesiumsulfat getrocknet und anschließend das Lösungsmittel im Vakuum abgezogen. Man erhält so 10 Fraktionen obiger Säure mit unterschiedlichem E/Z-Verhältnis. Das E/Z-Verhältnis wird durch das $^1$H-NMR-Spektrum bestimmt.

Fraktion I : (zuerst abgetrennte Säure) hat ein E/Z-Verhältnis von 85/15.

Fraktion IX : ein E/Z-Verhältnis von 40/60.

Fraktion IX (10,4 g) wird anschließend bei 30-40 °C in Cyclohexan gelöst. Durch stehenlassen bei Raumtemperatur kristallisiert (±)-trans-3-(Z-2-Chlor-2-(4-chlor-phenyl)-vinyl)-2,2-dimethyl-cyclopropancarbonsäure in Form farbloser Kristalle vom Schmelzpunkt 141-142 °C aus. Die Struktur wird durch das $^1$H-NMR-Spektrum bestätigt.

$^1$H-NMR-Spektrum in $CDCl_3$/TMS (ppm) :

aromatische-H : 2,37-2,81 (m/4 H), vinyl-H : 4,10 (d/1 H), cyclopropan-H : 7,26-7,56 (m/1 H) und 8,26 (d/1 H), dimethyl-H : 8,55 (s/3 H) und 8,70 (s/3 H).

(±) 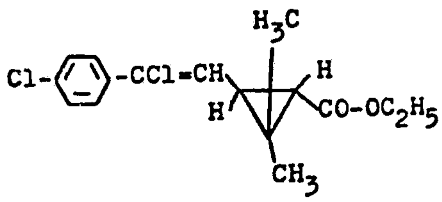

2,53 g (0,11 Mol) Natrium werden portionsweise in 50 ml Ethanol gelöst. Wenn sich alles Natrium aufgelöst hat, werden 150 ml Tetrahydrofuran (wasserfrei) zugesetzt und bei 0 °C unter Rühren 29,7 g (0,1 Mol) 4-Chlor-α-chlor-benzyl-phosphonsäure-diethylester, gelöst in 30 ml wasserfreiem Tetrahydrofuran, zugetropft. Nachdem noch 2 Stunden bei 0-5 °C nachgerührt wurde, werden 17 g (0,1 Mol) trans-2,2-Dimethyl-3-formyl-cyclopropancarbonsäureethylester, gelöst in 30 ml wasserfreiem Tetrahydrofuran, unter Rühren bei 0 °C zugetropft. Anschließend wird noch 12 Stunden bei 20-25 °C nachgerührt. Die Reaktionsmischung wird dann mit 500 ml Wasser versetzt un 2 mal mit je 300 ml Methylenchlorid extrahiert. Die organische Phase wird abgetrennt, über Magnesiumsulfat getrocknet, das Lösungsmittel im Wasserstrahlvakuum abdestilliert und der Rückstand im Vakuum destilliert. Man erhält 23,2 g (74,1 % der Theorie) (±)-trans-3-(E,Z-2-Chlor-2-(4-chlor-phenyl)-vinyl)-2,2-dimethyl-cyclopropancarbonsäure-ethylester als gelbes Öl mit dem Siedepunkt 155-165 °C/l Torr.

Beispiel

Test mit Boophilus microplus resistent

Lösungsmittel : 35 Gew.-Tle. Äthylenglykolmonomethyläther
35 Gew.-Tle. Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minuten getaucht. Nach Überführung in Plastikbecher und Aufbewahrung in einem klimatisierten Raum wird der Abtötungsgrad bestimmt.

Bei diesem Test zeigen die Diastereomeren des Beispiels 1 die in der folgenden Tabelle angegebenen Wirkungen ;

| Wirkstoff | Wirkstoffkonzentration in ppm | Abtötende Wirkung in % Boophilus microplus (OP-res. Biarra-St.) |
|---|---|---|
| Fraktion I des nach | 10 | 100 |
| dem Beispiel 1 her- | 3 | 100 |
| gestellten Wirkstoffs | 1 | > 50 |
| Variablenkombination | 0,3 | < 50 |
| [1](+)[2](Z)[3](R), | 0,1 | 0 |
| nicht beansprucht | 0,03 | 0 |
| | 0,01 | 0 |
| Fraktion II des nach | 10 | 100 |
| dem Beispiel 1 herge- | 3 | 100 |
| stellten Wirkstoffs | 1 | 100 |
| [1](+)[2](Z)[3](S) | 0,3 | > 50 |
| | 0,1 | > 50 |
| | 0,03 | < 50 |
| | 0,01 | 0 |

**Ansprüche**

1. Optisch aktive Isomere von trans-3-(2-Chlor-2-(4-chlor-phenyl)-vinyl)-2,2-dimethyl-cyclopropan-1-carbonsäure-(α-cyano-4-fluor-3-phenoxy-benzyl)-ester der allgemeinen Formel I

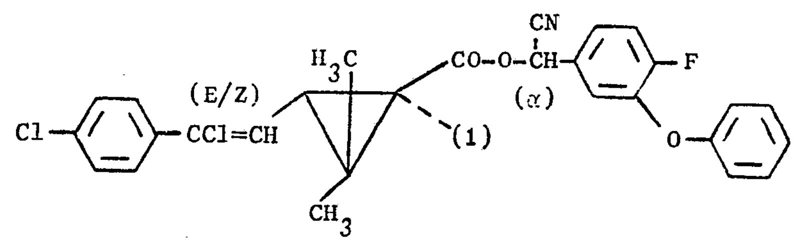

(I)

in der

(a) die Konfiguration am Cyclopropan-C-Atom (1) R oder S (Variable 1 = [1] ist, d. h. die den Verbindungen der Formel (I) zu Grunde liegenden Säuren drehen die Ebene des linear polarisierten Lichts nach rechts (+) bzw. nach links (−),

(b) die Konfiguration an der C-C-Doppelbindung E und/oder Z (Variable 2 = [2]) und

(c) die Konfiguration am α-C-Atom R und/oder S (Variable 3 = [3]) ist, gekennzeichnet durch die Variablenkombinationen [1] (+) [2] (E/Z) [3] (R/S), [1] (+) [2] (E) [3] (R/S) und [1] (+) [2] (Z) [3] (R/S) [1] (+) [2] (E/Z) [3] (S), [1] (+) [2] (E) [3] (S) und [1] (+) [2] (Z) [3] (S), [1] (−) [2] (E/Z) [3] (R/S), [1] (−) [2] (E) [3] (R/S), [1] (−) [2] (Z) [3] (R/S), [1] (−) [2] (E/Z) [3] (S), [1] (−) [2] (E) [3] (S) und [1] (−) [2] (Z) [3] (S), wobei die Symbole E und Z, (+) und (−) bzw. R und S bedeuten, daß die Isomeren der jeweils genannten Konfiguration auf mehr als 60 % angereichert sind und die Symbolkombinationen E/Z und R/S für Verbindungen der Formel I stehen, worin das Isomerenverhältnis zwischen 60 : 40 und 40 : 60 liegt.

2. Optisch aktive Isomere nach Anspruch 1, in denen die Symbole E und Z, (+) und (−) bzw. R und S bedeuten, daß die Isomeren der jeweils genannten Konfiguration auf mehr als 90 % angereichert sind.

3. Optisch aktive Isomere nach Anspruch 1, gekennzeichnet durch die Variablenkombinationen [1] (+) [2] (E/Z) [3] (R/S), [1] (+) [2] (E) [3] (R/S) und [1] (+) [2] (Z) [3] (R/S) [1] (+) [2] (E/Z) [3] (S), [1] (+) [2] (E) [3] (S) und [1] (+) [2] (Z) [3] (S).

4. Optisch aktive Isomere nach Anspruch 1, gekennzeichnet durch die Variablenkombinationen [1] (+) [2] (E/Z) [3] (R/S), [1] (+) [2] (E) [3] (R/S) und [1] (+) [2] (Z) [3] (R/S).

5. Verfahren zur Herstellung von Verbindungen der Formel I gemäß Anspruch 1, dadurch gekennzeichnet, daß man entsprechende optische aktive Isomere von trans-3-(2-Chlor-2-(4-chlorphenyl)-vinyl)-2, 2-dimethyl-cyclopropan-1-carbonsäurechlorid der Formel II

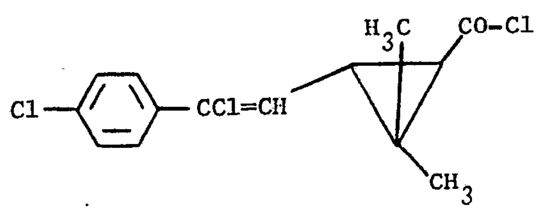

(II)

mit gegebenenfalls entsprechenden optischen Isomeren von α-Cyano-4-fluor-3-phenoxy-benzylalkohol der Formel III

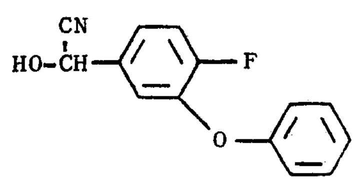

(III)

gegebenenfalls in Gegenwart eines Säureakzeptors und/oder in Gegenwart eines Verdünnungsmittels umsetzt oder, für den Fall, daß die Konfiguration am α-C-Atom R und S ein Verhältnis 1 : 1 ist, mit 4-Fluor-3-phenoxy-benzaldehyd der Formel IV

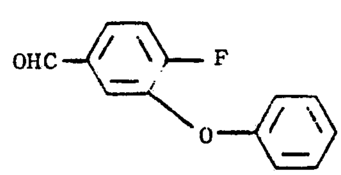

(IV)

in Gegenwart von wenigstens der äquimolaren Menge eines Alkalicyanids gegebenenfalls in Gegenwart

eines Katalysators und gegebenenfalls unter Verwendung von Verdünnungsmitteln umsetzt.

6. Ektoparasitizides Mittel, gekennzeichnet durch einen Gehalt an mindestens einer Verbindung der Formel I gemäß Anspruch 1.

7. Verwendung von Verbindungen der Formel (I) gemäß Anspruch 1 zur Bekämpfung von Ektoparasiten.

8. Verfahren zur Bekämpfung von Ektoparasiten, dadurch gekennzeichnet, daß man Verbindungen der Formel (I) gemäß Anspruch 1 auf Ektoparasitizide und/oder ihren Lebensraum einwirken läßt.

9. Optisch aktive Isomere von trans-3-(2-Chlor-2-(4-Chlorphenyl)-vinyl-2,2-dimethyl-cyclopropan-1-carbonsäuren und deren Säurehalogenide.

10. (+)-trans-3-(E/Z-2-Chlor-2-(4-chlor-phenyl)-vinyl-2,2-dimethyl-cyclopropan-1-carbonsäure, (+)-trans-3(E-2-Chlor-2-(4-chlor-phenyl)-vinyl-2,2-dimethyl-cyclopropan-1-carbonsäure und (+)-trans-3-(Z-2-Chlor-2-(4-chlor-phenyl)-vinyl)-2,2-dimethyl-cyclo-propan-1-carbonsäure, deren linksdrehende Isomere und deren Säurehalogenide

## Claims

1. Optically active isomers of trans-3-(2-chloro-2-(4-chloro-phenyl)-vinyl)-2,2-dimethyl-cyclopropane-1-carboxylic acid α-cyano-4-fluoro-3-phenoxy-benzyl ester of the general formula I

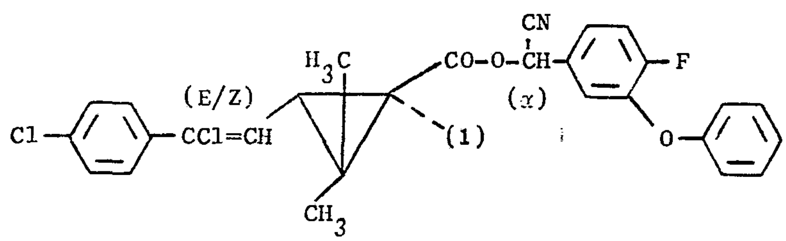

(I)

in which

(a) the configuration at the cyclopropane-C atom (1) is R or S (variable 1 = [1], that is to say the acids on which the compounds of the formula (I) are based rotate the plane of linearly polarised light to the right (+) or to the left (−),

(b) the configuration at the C-C double bond is E and/or Z (variable 2 = [2]) and

(c) the configuration at the α-C atom is R and/or S (variable 3 = [3]), which are characterised by the following combinations of variables : [1] (+) [2] (E/Z) [3] (R/S), [1] (+) [2] (E) [3] (R/S) and [1] (+) [2] (Z)[3] (R/S) [1] (+) [2] (E/Z) [3] (S), [1] (+) [2] (E)[3] (S) and [1] (+) [2] (Z) [3] (S) and [1] (−) [2] (E/Z) [3] (R/S), [1] (−) [2] (E) [3] (R/S), [1] (−) [2] (Z) [3] (R/S), [1] (−) [2] (E/Z) [3] (S), [1] (−) [2] (E) [3] (S) and [1] (−) [2] (Z) [3] (S), the symbols E and Z, (+) and (−) and R and S meaning that the compounds are enriched in the isomers of the particular configuration given to the extent of more than 60 %, and the symbol combinations E/Z and R/S representing compounds of the formula I wherein the isomer ratio is between 60 : 40 and 40 : 60.

2. Optically active isomers according to Claim 1, in which the symbols E and Z, (+) and (−) and R and S mean that the compounds are enriched in the isomers of the particular configuration given to the extent of more than 90 %.

3. Optically active isomers according to Claim 1, which are characterised by the following combinations of variables : [1] (+) [2] (E/Z) [3] (R/S), [1] (+) [2] (E) [3] (R/S) and [1] (+) [2] (Z) [3] (R/S) and [1] (+) [2] (E/Z) [3] (S), [1] (+) [2] (E) [3] (S) and [1] (+) [2] (Z) [3] (S).

4. Optically active isomers according to Claim 1, which are characterised by the following combinations of variables : [1] (+) [2] (E/Z) [3] (R/S), [1] (+) [2] (E) [3] (R/S) and [1] (+) [2] (Z) [3] (R/S).

5. Process for the preparation of compounds of the formula I according to Claim 1, characterised in that corresponding optically active isomers of trans-3-(2-chloro-2-(4-chloro-phenyl)-vinyl)-2,2-dimethyl-cyclopropane-1-carboxylic acid chloride of the formula II

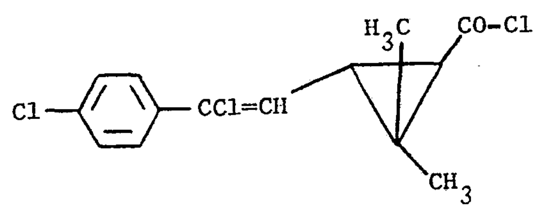

(II)

are reacted with, if appropriate, corresponding optical isomers of α-cyano-4-fluoro-3-phenoxy-benzyl alcohol of the formula III

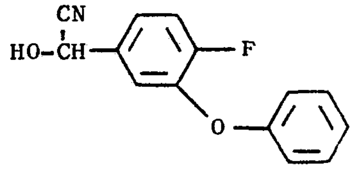

(III)

if appropriate in the presence of an acid acceptor and/or in the presence of a diluent, or, in the case where the configuration on the α-C atom is R and S in a ratio of 1 : 1, are reacted with 4-fluoro-3-phenoxybenzaldehyde of the formula IV

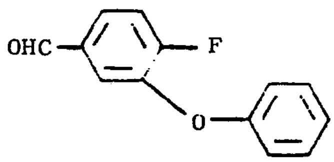

(IV)

in the presence of at least the equimolar amount of an alkali metal cyanide, if appropriate in the presence of a catalyst and if appropriate using diluents.

6. Ectoparasiticidal agent, characterised in that it contains at least one compound of the formula I according to Claim 1.

7. Use of compounds of the formula (I) according to Claim 1 for combating ectoparasites. 8. Process for combating ectoparasites, characterised in that compounds of the formula (I) according to Claim 1 are allowed to act on ectoparasiticides and/or their environment.

9. Optically active isomers of trans-3-(2-chloro-2-(4-chlorophenyl)-vinyl-2,2,-dimethyl-cyclopropane-1-carboxylic acids and acid halides thereof.

10. (+)-Trans-3-(E/Z-2-chloro-2-(4-chloro-phenyl)-vinyl-2,2-dimethyl-cyclopropane-1-carboxylic acid, (+)-trans-3-(E-2-chloro-2-(4-chloro-phenyl)-vinyl)-2,2-dimethylcyclopropane-1-carboxylic acid and (+)-trans-2-(Z-2-chloro-2-(4-chloro-phenyl)-vinyl)-2,2-dimethyl-cyclopropane-1-carboxylic acid, laevo-rotatory isomers thereof and acid halides thereof.

**Revendications**

1. Isomères optiquement actifs d'esters (α-cyano-4-fluoro-3-phénoxy-benzyliques) d'acides trans-3-(2-chloro-2-(4-chloro-phényl)-vinyl)-2,2-dimethyl-cyclopropane-1-carboxyliques de formule générale I

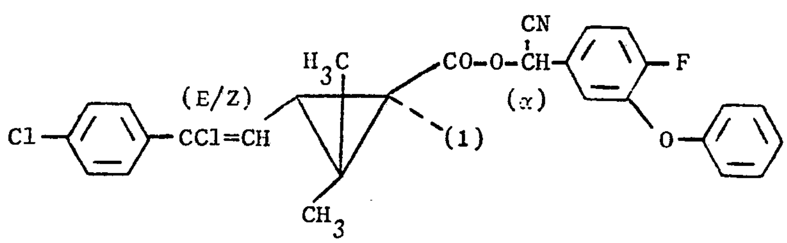

(I)

dans laquelle

(a) la configuration sur l'atome de carbone (1) du squelette cyclopropane est R ou S (variable 1 = [1]), c'est-à-dire que les acides à la base des composés de formule (I) font tourner le plan de la lumière linéaire polarisée vers la droite (+) ou vers la gauche (−),

(b) la configuration sur la double liaison C-C est E et/ou Z (variable 2 = [2]) et

(c) la configuration sur l'atome de carbone α est R et/ou S (variable 3 × [3]), caractérisés par les combinaisons de variables ci-après : [1] (+) [2] (E/Z) [3] (R/S), [1] (+) [2] (E) [3] (R/S) et [1] (+) [2] (Z) [3] (R/S) [1] (+) [2] (E/Z) [3] (S), [1] (+) [2] (E) [3] (S) et [1] (+) [2] (Z) [3] (S), [1] (−) [2] (E/Z) [3] (R/S), [1] (−) [2] (E) [3] (R/S), [1] (−) [2] (Z) [3] (R/S), [1] (−) [2] (E/Z) [3] (S), [1] (−) [2] (E) [3] (S) et [1] (−) [2] (Z) [3] (S), les symboles E et Z, (+) et (−) ou R et S signifiant que les isomères de chaque configuration mentionnée sont concentrés à plus de 60 %, tandis que les combinaisons de symboles E/Z et R/S indiquent des composés de formule I où le rapport des isomères se situe entre 60 : 40 et 40 : 60.

2. Isomères optiquement actifs suivant la revendication 1, dans lesquels les symboles E et Z, (+) et (−) ou R et S signifient que les isomères de chaque configuration mentionnée sont concentrés à plus de 90 %.

3. Isomères optiquement actifs suivant la revendication 1, caractérisés en ce qu'ils se présentent sous les combinaisons de variables ci-après : [1] (+) [2] (E/Z) [3] (R/S), [1] (+) [2] (E) [3] (R/S) et [1] (+) [2] (Z) [3] (R/S) [1] (+) [2] (E/Z) [3] (S), [1] (+) [2] (E) [3] (S) et [1] (+) [2] (Z) [3] (S).

4. Isomères optiquement actifs suivant la revendication 1, caractérisés en ce qu'ils se présentent sous les combinaisons de variables ci-après : [1] (+) [2] (E/Z) [3] (R/S), [1] (+) [2] (E) [3] (R/S) et [1] (+) [2] (Z) [3] (R/S).

5. Procédé de préparation de composés de formule I suivant la revendication 1, caractérisé en ce qu'on fait réagir des isomères optiquement actifs correspondants du chlorure d'acide trans-3-(2-chloro-2-(4-chlorophényl)-vinyl)-2,2-dimethyl-cyclopropane-1-carboxylique de formule II :

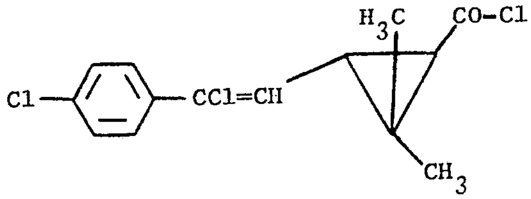

(II)

avec des isomères optiques éventuellement correspondants à l'alcool α-cyano-4-fluoro-3-phénoxy-benzylique de formule III :

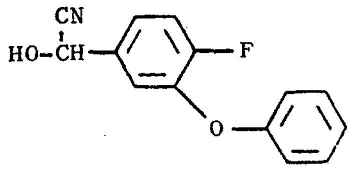

(III)

éventuellement en présence d'un accepteur d'acide et /ou en présence d'un diluant ou, si la configuration sur l'atome de carbone α est R et S dans un rapport de 1 : 1, avec le 4-fluoro-3-phénoxy-benzaldéhyde de formule IV :

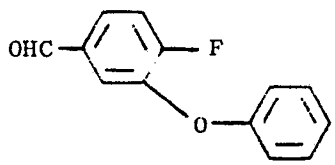

(IV)

en présence d'au moins la quantité équimolaire d'un cyanure alcalin, éventuellement en présence d'un catalyseur et éventuellement en utilisant des diluants.

6. Agent ectoparasiticide, caractérisé en ce qu'il contient au moins un composé de formule I suivant la revendication 1.

7. Utilisation de composés de formule (I) suivant la revendication 1 pour combattre les ectoparasites.

8. Procédé en vue de combattre les ectoparasites, caractérisé en ce qu'on fait agir des composés de formule (I) suivant la revendication 1 sur des ectoparasites et/ou sur leur biotope.

9. Isomères optiquement actifs d'acides trans-3-(2-chloro-2-(4-chlorophényl)-vinyl)-2,2-diméthyl-cyclopropane-1-carboxyliques et de leurs halogénures d'acides.

10. L'acide (+)-trans-3-(E/Z-2-chloro-2-(4-chlorophényl)-vinyl)-2,2-diméthyl-cyclopropane-1-carboxylique, l'acide (+)-trans-3-(E-2-chloro-2-(4-chloro-phényl)-vinyl)-2,2-diméthyl-cyclopropane-1-carboxylique et l'acide (+)-trans-3-(Z-2-chloro-2-(4-chloro-phényl)-vinyl)-2,2-diméthyl-cyclopropane-1-carboxylique, leurs isomères lévogyres et leurs halogénures d'acides.